# EUROPEAN PATENT APPLICATION

(11) **EP 2 065 002 A1**
(43) Date of publication of application: **03.06.2009**
(21) Application number: 08478001.4
(22) Date of filing: 21.11.2008
(51) Int. Cl.: A61B 17/22

(54) **Ultrasound wave guide wire for internal blood vessels cleaning**

(30) Priority: 28.11.2007 LT 2007074
(71) Applicant: Kauno Technologijos Universitetas, 44029 Kaunas (LT)
(72) Inventor: Bansevicius, Ramutis, 51067 Kaunas (LT); Bubulis, Algimantas, 52177 Kaunas (LT); Jurenas, Vytautas, 52177 Kaunas (LT); Minchenia, Vladimir, Minsk (BY); Valaika, Marius, 50143 Kaunas (LT)
(74) Representative: Sidlauskiene, Aurelija

(57) **Abstract**

The invention is assigned to the field of medicine and may be used for the internal blood vessel walls cleaning.

In case of increasing blood vessels cleaning efficiency and reliability, in ultrasound wave guide wire used for the internal blood vessel cleaning, consisting of the metal wire with tapered flexible cross-section 1 and working part 2, which is in the distal end, the wire is produced of flexible material, distal wire part equally makes the working part 2, which is of spiral shape and its length is not less than ¼ λ, where λ is wave guide wire ultrasound longitudinal vibration wave length and strand step is not more than 0,5 mm and spiral end makes closed circuit, which is formed when the spiral last strand ending connected with the last strand beginning forms the closed ring shaped circuit 3, when the spiral last strand ending through all spiral internal length is connected with the spiral first strand beginning by the general element 4 and the case when the spiral is tube shaped, which walls are flexed in zigzag and the last zigzag strand ending is connected to the last zigzag strand beginning. Also when the flexible guide wire 5 may be entered through the internal working part slot and the working part may go through arterial vessels and flex into different directions. Furthermore, all the spiral part connections are produced by one or both mechanical twisting and spot welding manners.

## Description

The invention is assigned to the field of medicine and may be used for the internal blood vessel walls cleaning.

It is known catheter-based ultrasound delivery system consists of ultrasound power generator connected to the piezoelectric transducer through the horn attached to the proximal end of the metal wire probe which distal end is of spiral shape. The horn and wire probe which distal end is of spiral shape are produced of the same high elasticity material, such as stainless steel, nickel, titanium or its alloy (see LT patent Nr. 5248, A61 B 17/20, 2005).

In the specified ultrasound catheter the metal wire probe end of the spiral shape is free and because of it the breakage of spiral part is possible at some vibration frequencies and this complicates the invasive therapeutics process and reduces reliability.

The ultrasound internal blood vessel walls cleaning device is known and it consists of wave guide wire, vibrating at ultrasound frequency which is connected with thin elements which are vibrating in their own low frequency resonant modes (DE10146011 A1, A61B17/22, 2003).

In the mentioned device, the wave guide wire construction does not allow reaching the sufficient amplitude of the working element and does not secure the internal blood vessel walls against perforation, thrombus that may stuff the smaller blood vessels, are chopped up in big fractions as well.

The aim of this invention is to increase blood vessels cleaning efficiency and reliability.

The named aim is accessible because of that in the ultrasound wave wire for the internal blood vessel cleaning, consisting of wire with uniform flexible cross-section and working part in the distal end, the wire is made of flexible material, distal wire part equally makes the working part, which is of spiral shape and its length is not less than ¼ λ, where λ is wave length of the wave guide wire ultrasound longitudinal vibration, strand step is not more than 0,5 mm and spiral end makes closed circuit.

Also the spiral end closed circuit is kept in cases when the spiral last strand end at the whole spiral internal length is connected with the first strand beginning by the general element and the spiral is tube shaped, which walls are flexed in zigzag and the last zigzag strand ending is connected to the last zigzag strand beginning.

Also through the internal working part slot, which is spiral shaped, it is possible to enter flexible guide wire, the working part may go through arterial vessels and flex into different directions and all spiral joining parts are produced with one or both mechanical twisting and spot welding manners.

The invention is described in drawings.
1. Fig. Ultrasound wave guide wire which working part is spiral shaped and the free ending is connected to the last strand beginning and makes closed, ring shaped circuit is presented in the first picture.
2. Fig. Ultrasound wave wire which spiral's last strand ending, through all the internal spiral length connected to spiral's first strand beginning is presented in the second picture.
3. Fig. Ultrasound wave wire which spiral is tube shaped and its walls are flexed in zigzag is presented in the third picture.
4. Fig. Ultrasound wave wire in which the flexible guide wire is entered through spiral's internal part is presented in the fourth picture.

The ultrasound wave guide wire used for the internal blood vessel walls cleaning consists of the metal wire with tapered form segment 1 and working segment 2, which is spiral shaped and its ending forms closed ring shaped 3 form circuit. Otherwise, the spiral's last strand ending through all the internal spiral length is connected with the spiral's first strand beginning by general element 4. The flexible guide wire 5 may be entered through spiral internal part and it directs the wave guide wire working part 2 distal end into the thrombus forming place in blood vessel.

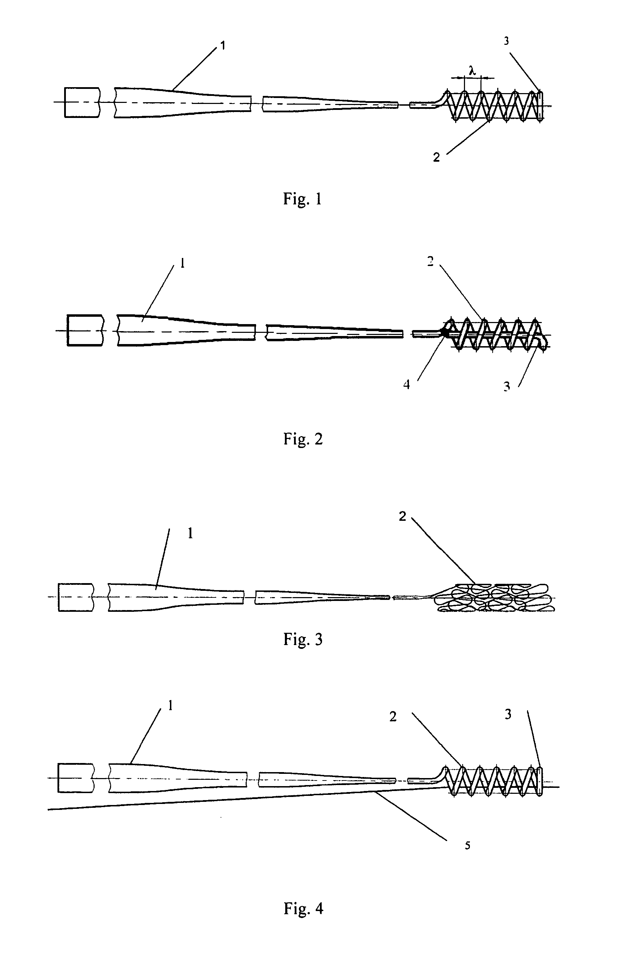

The device is handled in such way:
High frequency mechanic vibrations are transmitted from the piezoelectric transducer (not shown in the drawing) through tapered metal wave guide wire into the working part 2, which is spiral shaped. The wire is flexible and has tapered form, and because of it; it functions as a horn intensifying ultrasound mechanical vibrations. Concentrated ultrasound energy is transmitted to spiral shape segment through the metal wire, which being in water erases cavitations processes (i.e. in the vessels blood). Cavitations process spreads through all spiral length in radial direction and at spiral ending - in axial direction. The cavitation process results formation of the micro bubbles in the fluid (i.e. blood), which explosion energy disrupts the blood clots and destroy (eliminate) thrombus of the vessel. In case when the spiral last strand ending through internal spiral length, general element 4 is connected with the spiral first strand beginning, energy concentration spreads equivalently through all spiral length and results the disintegration of the thrombus into tiny particles. In all spiral form changing cases the closed ring at the ending does not let the perforation of the internal blood vessels walls and the zigzag shaped additionally collects micro clots into the spiral inside that while pulling out the wave guide wire they are eliminated from the blood vessel. Also the chosen wire length, which is not longer than ¼ of a wave length floating through the wave wire, lets to reach the maximum of the working amplitude not enlarging the energy source excitation. The distance between the spiral strands must be not more than 0,5mm and it makes the best favorable terms to create the cavitations process in the radial direction.
As compared with the prototype, the ultrasound wave guide wire with new constructive elements set when the appropriate spiral shape is chosen, their length and strand step and forming the spiral ending into closed circuit, reduces the risk of blood vessel perforation and chops the blood vessel thrombus into tiny dispersal particles, not causing the new thrombus formation danger and this enlarges the blood vessels cleaning reliability and efficiency.

## Claims

1. The ultrasound wave wire used for the internal blood vessel cleaning consists of the metal wave guide wire with flexible tapered segment and working part, which is in the distal end, **characterized in that** the wire is made of flexible material, distal wire part equivalently makes the working part, which is spiral shaped, which length is not less than ¼ λ, where λ is wave guide wire ultrasound longitudinal vibration wave length and strand step is not more than 0,5 mm and spiral end makes closed circuit.

2. The ultrasound wave wire used for the internal blood vessel cleaning according to claim 1, **characterized in that** the spiral last strand ending connected with the last strand beginning and forms the closed ring shaped circuit.

3. The ultrasound wave wire used for the internal blood vessel cleaning according to claim 1 **characterized in that** the spiral last strand ending through all spiral internal length is connected with the spiral first strand beginning by the general element.

4. The ultrasound wave wire used for the internal blood vessel cleaning according to claim 1, **characterized in that** the spiral is tube shaped, which walls are flexed in zigzag and the last zigzag strand ending is connected to the last zigzag strand beginning forming the closed circuit.

5. The ultrasound wave wire used for the internal blood vessel cleaning according to claims 1, 2, 3, 4, **characterized in that** the flexible guide wire may be entered through the internal working part slot, which is spiral shaped.

6. The ultrasound wave wire used for the internal blood vessel cleaning according to claim 1, **characterized in that** the working part may go through arterial vessels and flex into different directions.

7. The ultrasound wave wire used for the internal blood vessel cleaning according to claim 1, **characterized in that** the spiral part connections produced by one or both mechanical twisting and spot welding manner
